# EUROPEAN PATENT SPECIFICATION

(11) **EP 3 810 254 B1**
(45) Date of publication and mention of the grant of the patent: **21.02.2024**
(21) Application number: 19727017.6
(22) Date of filing: 29.05.2019
(51) Int. Cl.: A61M 39/10, A61M 5/31

(54) **CONNECTOR ASSEMBLY FOR CONNECTING MEDICAL LINES COMPRISING A CAP ELEMENT**
VERBINDERANORDNUNG ZUM VERBINDEN MEDIZINISCHER LEITUNGEN MIT EINEM KAPPENELEMENT
ENSEMBLE RACCORD DESTINÉ À RACCORDER DES TUYAUX MÉDICAUX COMPRENANT UN ÉLÉMENT DE RECOUVREMENT

(30) Priority: 20.06.2018 EP 18178716
(43) Date of publication of application: 28.04.2021
(73) Proprietor: Fresenius Kabi Deutschland GmbH, 61352 Bad Homburg (DE)
(72) Inventor: BAUMGART, Steffen, 36088 Hünfeld (DE); FISCHER, Dirk, 99831 Creuzburg (DE)
(74) Representative: Fresenius Kabi Deutschland GmbH
(86) International application number: PCT/EP2019/063935
(87) International publication number: WO 2019/243011

(56) References cited:
- WO-A1-2014/159346
- WO-A1-2017/095373
- DE-A1-102011 013 792

## Description

The invention relates to a connector assembly for connecting medical lines to each other according to the preamble of claim 1. The invention is defined in claim 1. Further embodiments of the invention are defined in the dependent claims.

A connector assembly of this kind comprises a first connector having an insertion shaft to be inserted, along an insertion direction, into an insertion opening of a second connector for forming a fluid connection between the medical lines, the insertion shaft comprising a fluid channel allowing fluid to flow in between the first connector and the second connector when the first connector and the second connector are attached to each other.

A medical line of this kind may for example be a line used for the enteral feeding of a patient. Enteral feeding generally refers to the delivery of a nutritionally complete feed, containing protein, carbohydrate, fat, water, minerals and vitamins, directly into the stomach, duodenum or jejunum of a patient. A feeding tube, for this purpose, may for example be passed through the nares (nostril), down the esophagus and into the stomach (so-called nasogastric feeding tube). A nasojejunal feeding tube, in comparison, may be passed further through the stomach into the jejunum, the middle section of the small intestine. And a gastric feeding tube is a tube inserted through a small incision in the abdomen into the stomach and is used, preferably, for long-term enteral nutrition.

When using feeding lines for the enteral feeding of a patient, a first line may for example be placed on the patient, for example providing a feeding port to the stomach. This first line may remain on the patient for a rather long time, for example a few days or even a few weeks. Another, second line may be connected to this first line for the actual feeding wherein for each feeding procedure a new second line may be used and disposed after the feeding procedure.

A connector assembly in this example serves to connect the feeding lines to each other. A first connector (also denoted as male connector, for example a so-called ENFit male connector) herein may be arranged for example on the line placed on the patient, whereas a second connector (female connector, for example a so-called ENFit female connector) may be used on the second line for connecting the second line to the first line for carrying out an actual feeding procedure.

Whereas the second connector may be used only once for carrying out a feeding procedure, the first connector connected for example to a line placed on a patient may be re-used multiple times. Because the first connector may be re-used multiple times for multiple feeding procedures, it is to be made sure that the first connector is clean when connecting the lines to each other, in order to avoid a contamination of any solution passed towards the patient. Furthermore, no fluid carried out of the first connector if it is not connected to a corresponding second connector.

Therefore, cap elements are used to close of the first connector. In WO 2017/095373 A1 a connector assembly comprising a first connector and a cap element is disclosed. The first connector is a male connector having an outer locking collar and an internal tapered sealing wall. The cap element is configured to receive a tapered sealing wall of the male connector so as to form a seal with an outer surface of the tapered sealing wall.

It is an object of the instant invention to provide a connector assembly which allows to keep the first connector clean as well as to prevent a fluid leakage out of the first connector when no second connector is connected to the first connector.

This object is achieved by a connector assembly comprising the features of claim 1.

Accordingly, the connector assembly comprises a cap element attachable to the first connector for closing the fluid channel towards the outside in a state in which the first connector is not connected to the second connector, the first connector having a first retention device and the cap element having a second retention device, the first retention device and the second retention device being configured to form a positive locking engagement in between the cap element and the first connector along the insertion direction.

Hence, a cap element can be attached to the first connector, the cap element serving to close the fluid channel formed in the insertion shaft towards the outside when no second connector is connected to the first connector. The cap element can be attached to the first connector, and when it is attached to the first connector a contamination of the fluid channel formed in the insertion shaft as well as a leakage of fluid out of the fluid channel is prevented.

The cap element may be formed for example from a soft, elastic material, such as a plastics material, for example an elastomer, or a rubber material.

In order to retain the cap element on the first connector, a positive locking engagement in between the cap element and the first connector is formed when the cap element is attached to the first connector. For this, the cap element as well as the first connector each comprise a retention device, which act together when the cap element is attached to the first connector for forming the positive locking engagement in between the cap element and the first connector.

The positive locking engagement acts along the insertion direction. Namely, the cap element can be attached to the first connector by placing it on the first connector along the insertion direction. Once it is attached to the first connector the positive locking engagement in between the retention devices of the cap element and the first connector is formed, such that the cap element cannot be removed from the first connector along the insertion direction without releasing the positive locking engagement of the retention devices.

In one embodiment, the first retention device is formed within the fluid channel of the insertion shaft. The first retention device may for example be formed as a recess in a wall surrounding the fluid channel, the second retention device of the cap element being configured to engage with the first retention device in the shape of the recess once the cap element is attached to the first connector.

The first connector may for example be formed from a plastics material by using an injection molding technique. The fluid channel herein is integrally formed within the insertion shaft, wherein the recess within the fluid channel is formed by a portion of the fluid channel having a wider radius than another portion such that a stop face is formed within the fluid channel which may engage with the other, second retention device of the cap element.

Herein, to ease the manufacturing of the first connector by using an injection molding technique the recess may extend within the fluid channel towards and up to an end of the first connector opposite to the insertion shaft with which the first connector may be connected to the second connector. In this way the recess may integrally be formed in an easy way when forming the first connector by injection molding.

In one embodiment, the cap element comprises a cap head which, when the cap element is attached to the first connector, covers the fluid channel formed in the insertion shaft towards the outside. The second retention device herein may protrude from the cap head along the insertion direction such that the second retention device may for example engage with the first retention device formed as a recess within the fluid channel of the first connector.

In one embodiment, the second retention device comprises one or multiple retention heads at an end opposite to the cap head. Each retention head for example forms a radially protruding edge via which the positive locking engagement in between the cap element and the first connector is established when the cap element is attached to the first connector. By means of one or multiple retention heads the cap element in particular may engage with the first retention device of the first connector formed as a recess within the fluid channel such that by means of the engagement the cap element is held on the first connector along the insertion direction.

The insertion shaft may for example have a cylindrical or conical shape having a circular cross section. The insertion opening of the second connector has a complementary cylindrical or conical shape and hence may receive the insertion shaft upon connecting the connectors to each other. The fluid channel of the first connector herein extends through the insertion shaft along the insertion direction such that via the fluid channel a fluid connection in between the first connector and the second connector may be established when the second connector is connected to the first connector.

The first connector, in one embodiment, comprises a first body circumferentially extending about the insertion shaft such that an inner space is formed radially in between the first body and the insertion shaft. The first body hence is placed at a radial distance to the insertion shaft and is arranged for example coaxially with the insertion shaft. In between the first body and the insertion shaft the radial inner space is formed in which, preferably, an insertion portion of the cap element is received when the cap element is attached to the first connector. The insertion portion may for example have a generally cylindrical or conical shape and serves to engage with the inner space of the first connector such that the inner space of the first connector is at least partially filled when the cap element is attached to the first connector.

The insertion portion of the cap element may for example be arranged concentrically (with respect to the insertion direction) with the second retention device. The insertion portion may herein be arranged radially outside of the second retention device and may circumferentially extend about the second retention device. The second retention device hence may engage with the first retention device formed for example within the fluid channel of the insertion shaft when attaching the cap element to the first connector, whereas the insertion portion may reach into the inner space formed around the insertion shaft and hence may encompass the insertion shaft when the cap element is attached to the first connector.

In one embodiment the cap element, in particular by its cap head, may be configured to close the inner space formed in between the insertion shaft and the first body circumferentially extending about the insertion shaft towards the outside such that a contamination of the first connector, in particular the inner space of the first connector, from the outside is prevented when the cap element is attached to the first connector. In addition, because the cap element closes the fluid channel formed in the insertion shaft, no fluid may leak out of the fluid channel, such that no fluid from the fluid channel may enter into the inner space and may not contaminate the inner space of the first connector.

The connectors may for example be connected to each other by means of a screw connection. Herein, for example on an inner face of the first body a first screw thread may be provided, the first screw thread being engageable with a second screw thread on a second body of the second connector. Hence, for connecting the connectors to each other, the connectors are screwed together, such that the insertion shaft of the first connector is inserted along the insertion direction in the corresponding insertion opening of the second connector.

The second connector beneficially comprises a second body in which the insertion opening for receiving the insertion shaft of the first connector is formed. On the circumferential outer wall of the second body a second screw thread may be formed and may be engageable with the first screw thread of the first connector for establishing a screw type connection in between the connectors.

The connector assembly provides for the connection between lines, for example feeding lines for the enteral feeding. Correspondingly, the first connector may comprise a first connection shaft axially aligned with the insertion shaft for receiving a first connection line, whereas the second connector may comprise a second connection shaft axially aligned with the insertion opening for receiving a second connection line. Hence, connection lines, such as feeding lines, may be connected to the connectors and, via the connectors, may be connected to each other to provide for a liquid transfer between the connection lines.

The idea underlying the invention shall subsequently be explained in more detail with reference to the embodiments shown in the figures. Herein:
- Fig. 1: shows a schematic drawing of a feeding line placed on a patient;
- Fig. 2: shows an embodiment of a connector assembly having a first connector and a second connector; and
- Fig. 3: shows an embodiment of a first connector comprising a cap element.

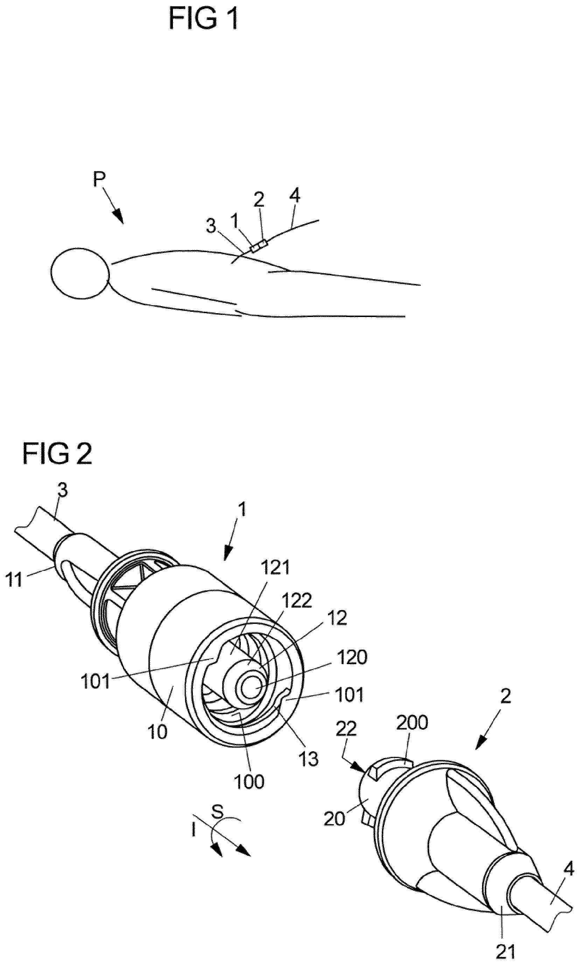

Fig. 1 shows a schematic drawing of a feeding line assembly comprising feeding lines 3, 4 placed on a patient P.

The feeding lines 3, 4 for example serve for providing for an enteral feeding of the patient P. For this, a first line 3 may, for example through an incision in the abdomen of the patient P, be inserted into the stomach of the patient P and may be used as a port for a long-term enteral feeding. A second feeding line 4 is connected to the first feeding line 3 via a connector assembly comprising connectors 3, 4, the second feeding line 4 for example being connected to a container comprising an enteral feeding solution, which via the lines 3, 4 is delivered towards the patient P for example using a suitable pumping device.

Whereas the second feeding line 4 may be used for example only once in connection with the container comprising the enteral feeding solution and may be disposed after use, the feeding line 3 placed on the patient P may remain on the patient P for a rather long term, for example a few days or even a few weeks, and may be used multiple times to carry out multiple feeding procedures. There hence is a necessity to keep the feeding line 3 as well as the connector 1 connected to the feeding line 3 clean, such that a contamination of the feeding line 3 and the connector 1 connected to it is effectively prevented.

An embodiment of a connector assembly comprising connectors 1, 2 is shown in Fig. 2. The first connector 1 herein may for example be connected to the feeding line 3 placed on the patient P, whereas the second connector 2 is arranged on the other, second feeding line 4 connected to a container comprising a feeding solution.

The first connector 1, in this embodiment, comprises a cylindrical body 10 circumferentially coaxially extending about a cylindrical or conical insertion shaft 12 and forming, together with the insertion shaft 12, a radial inner space 13. On the inner wall of the body 10 a screw thread 100 is arranged allowing for a screw type connection with the second connector 2.

The insertion shaft 12 can be inserted in an insertion direction I into an insertion opening 22 in a body 20 of the second connector 2. On the body 20 of the second connector 2 a screw thread 200 is arranged, such that the second connector 2 can be screwed into the inner space 13 of the first connector 1 by engaging the screw threads 100, 200 with each other and by screwing the first connector 1 along a screwing direction S on the second connector 2.

The insertion shaft 12 has a substantially cylindrical or conical shape having a circular cross section. The insertion opening 22 of the second connector 2 has a corresponding, complementary shape and hence is suited to receive the insertion shaft 12.

When screwing the connectors 1, 2 on to each other, the insertion shaft 12 is inserted into the insertion opening 22. The insertion shaft 12 comprises a fluid channel 120, which extends through the insertion shaft 12 and through a shaft 11 aligned with the insertion shaft 12, the shaft 11 being connected to the associated feeding line 3. Likewise, the insertion opening 22 is aligned with a shaft 21 of the second connector 2, the shaft 21 being connected to the feeding line 4. Hence, by connecting the connectors 1, 2 to each other, a fluid connection between the feeding lines 3, 4 can be established, such that a medical fluid such as an enteral feeding solution may pass from the feeding line 4 through the connectors 1, 2 into the feeding line 3.

The connectors 1, 2 each may for example be fabricated from a comparatively hard plastics material, for example by injection molding.

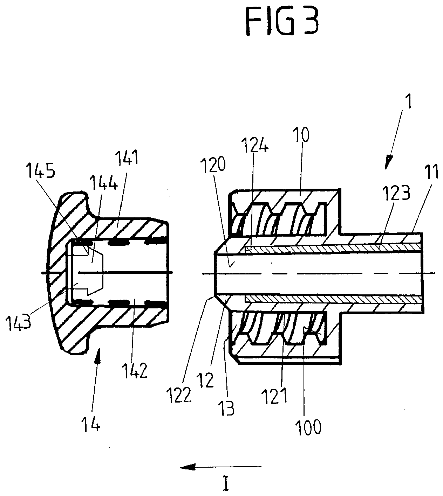

In a state in which the second connector 2 is not arranged on the first connector 1, it is desirable to prevent a contamination of the first connector 1 from the outside as well as a fluid leakage from the fluid channel 120 formed in the insertion shaft 12 of the first connector 1. For this, in the embodiment of Fig. 3 a cap element 14 is provided which for example may be formed from a soft elastic material, such as a soft plastics material, for example an elastomer, or a rubber material. The cap element 14 may be placed on the first connector 1 along the insertion direction I such that, when it is attached to the first connector 1, it closes the first connector 1, in particular the fluid channel 120 formed within the first connector 1 towards the outside.

The cap element 14 comprises an insertion portion 141 protruding from a cap head 140 and being integrally formed with the cap head 140. The insertion portion 141 may be introduced into the space 13 formed radially in between the insertion shaft 12 and the body 10 of the first connector 1 such that the space 13 is at least partially filled by means of the insertion portion 141 if the cap element 14 is attached to the first connector 1.

Radially inside the insertion portion 141 of the cap element 14 an opening is formed which serves to receive the insertion shaft 12 when the cap element 14 is attached to the first connector 1. The cap element 14 comprises a retention device 143 in the shape of a pin carrying a retention head 144 forming a circumferentially surrounding, protruding locking protrusion 145, the retention device 143 protruding from the cap head 140 into the opening formed by the insertion portion 141 along the insertion direction I.

The retention device 143, in the shown embodiment, is rotationally symmetric, the locking protrusion 145 extending circumferentially about the retention head 144. The retention device 143 herein is concentrical with the insertion portion 141, the insertion portion 141 circumferentially encompassing the retention device 143.

The retention device 143 of the cap element 14 serves to engage with a retention device 123 formed by a recess within the fluid channel 120 of the first connector 1. The retention device 123 in the shape of the recess is formed by a radial widening of the fluid channel 120, the recess 123 forming a stop face 124 in the vicinity of the outer end of the insertion shaft 120. In particular, the stop face 124 forms a step with respect to a wall 125 surrounding the fluid channel 120 in the vicinity of the outer end of the insertion shaft 12.

When the cap element 14 is attached on the first connector 1, the retention device 143 with its retention head 144 is plugged into the fluid channel 120 and comes into engagement with the retention device 123 formed as a recess within the fluid channel 120 such that the locking protrusion 145 abuts the stop face 124 and a positive locking engagement in between the cap element 14 and the first connector 1 is established. The cap element 14 hence is held on the first connector 1 such that it may not easily fall off the first connector 1.

Because the cap element 14 may beneficially be formed from a soft elastic material, a user may remove the cap element 14 from the first connector 1 by pulling it off the first connector 1 in the insertion direction I. The retention device 123 is integrally formed with the cap element 14 and is also formed from a soft elastic material, such that the retention head 144 may deform for releasing the locking engagement with the retention device 123 in the shape of the recess formed in the fluid channel 120.

In one embodiment, the retention device 123 in the shape of the recess is only locally formed within the fluid channel 120. The retention device 123 herein is formed at the axial location at which a positive locking engagement in between the retention device 143 of the cap element 14 and the retention device 123 of the first connector 1 is to be established.

In another embodiment, the retention device 123 may extend all the way through the shaft 11 adjoining the body 10, as this is shown in the embodiment of Fig. 3. This may ease the manufacturing of the first connector 1 by means of an injection molding technique because the retention device 123 in the shape of the recess may easily be formed within an injection molding tool.

If a second connector 2 is to be connected to the first connector 1, the cap element 14 may be removed from the first connector 1 such that the second connector 2 may be arranged on the first connector 1 establishing a screw connection in between the connectors 1, 2. If the second connector 2 is again removed, the cap element 14 (or another, un-used cap element 14) may be once more placed on the first connector 1.

The invention is not limited to the embodiments described above, but may be implemented in an entirely different fashion.

For example, a connector assembly of the kind described herein may not only be used for the enteral feeding, but may be used for connecting any medical lines to each other for transferring medical solutions, such as medication, nutritional solutions, saline solutions or any other solutions.

### List of Reference Numerals

- 1: Connector (male part)
- 10: Body
- 100: Screw thread
- 101: Protrusion
- 11: Shaft
- 12: Insertion shaft
- 120: Fluid channel
- 121: Surface (outer wall)
- 122: Tip
- 123: First retention device (recess)
- 124: Stop face
- 125: Wall
- 13: Space
- 14: Cap element
- 140: Cap head
- 141: Insertion portion
- 142: Inner space
- 143: Second retention device
- 144: Retention head
- 145: Locking protrusion
- 2: Connector (female part)
- 20: Body
- 200: Screw thread
- 21: Shaft
- 22: Insertion opening
- 3: Line
- 4: Line
- I: Insertion direction
- P: Patient
- S: Screwing direction

## Claims

1. Connector assembly for connecting medical lines (3, 4) to each other, comprising: a first connector (1) having an insertion shaft (12) to be inserted, along an insertion direction (I), into an insertion opening (22) of a second connector (2) for forming a fluid connection between the medical lines (3, 4), the insertion shaft (12) comprising a fluid channel (120) allowing fluid to flow in between the first connector (1) and the second connector (2),
a cap element (14) attachable to the first connector (1) for closing the fluid channel (120) towards the outside in a state in which the first connector (1) is not connected to the second connector (2), the first connector (1) having a first retention device (123) and the cap element (14) having a second retention device (143), the first retention device (123) and the second retention device (143) being configured to form a positive locking engagement in between the cap element (14) and the first connector (1) along the insertion direction (I),
**characterized in**
**that** the first retention device (123) is formed as a recess in a wall (125) surrounding the fluid channel (120).

2. Connector assembly according to claim 1, **characterized in that** the first retention device (123) is formed within the fluid channel (120) of the insertion shaft (12).

3. Connector assembly according to one of claims 1 to 2, **characterized in that** the cap element (14) comprises a cap head (140), the second retention device (143) protruding from the cap head (140) along the insertion direction (I).

4. Connector assembly according to one of the preceding claims, **characterized in that** the second retention device (143) comprises at least one retention head (144) at an end opposite to the cap head (140), the at least one retention head (144) being formed to form said positive locking engagement with the first retention device (123).

5. Connector assembly according to one of the preceding claims, **characterized in that** the insertion shaft (12) has a cylindrical or conical shape, the fluid channel (120) extending along the insertion direction (I) in the insertion shaft (12).

6. Connector assembly according to one of the preceding claims, **characterized in that** the first connector (1) comprises a first body (10) circumferentially extending about the insertion shaft (12) such that an inner space (13) is formed radially in between the first body (10) and the insertion shaft (12), the cap element (14) comprising an insertion portion (141) insertable into the inner space (13).

7. Connector assembly according to claim 6, **characterized in that** the insertion portion (141) is arranged concentrically with the second retention device (143).

8. Connector assembly according to claim 6 or 7, **characterized in that** the insertion portion (141) is arranged radially outside of the second retention device (143).

9. Connector assembly according to one of claims 6 to 8, **characterized in that** the cap element (14) is configured to close the inner space (13) towards the outside when the cap element (14) is attached to the first connector (1).

10. Connector assembly according to one of claims 6 to 9, **characterized in that** the first body (10) comprises a screw thread (100) for connecting the first connector (1) and the second connector (2) to each other.

11. Connector assembly according to one of the preceding claims, **characterized in that** the first connector (1) comprises a first connection shaft (11) axially aligned with the insertion shaft (12) for receiving a first connection line (3).

## Patentansprüche

1. Verbinderanordnung zum Miteinanderverbinden von medizinischen Leitungen (3, 4), die Folgendes umfasst: einen ersten Verbinder (1) mit einer Einsteckwelle (12), die zum Bilden einer Fluidverbindung zwischen den medizinischen Leitungen (3, 4) entlang einer Einsteckrichtung (I) in eine Einstecköffnung (22) des zweiten Verbinders (2) einzustecken ist, wobei die Einsteckwelle (12) einen Fluidkanal (120) umfasst, der es einem Fluid erlaubt, zwischen dem ersten Verbinder (1) und dem zweiten Verbinder (2) zu fließen,
ein Deckelelement (14), das in einem Zustand, in dem der erste Verbinder (1) nicht mit dem zweiten Verbinder (2) verbunden ist, zum Schließen des Fluidkanals (120) zur Außenseite am ersten Verbinder (1) befestigbar ist, wobei der erste Verbinder (1) eine erste Haltevorrichtung (123) aufweist und das Deckelelement (14) eine zweite Haltevorrichtung (143) aufweist, wobei die erste Haltevorrichtung (123) und die zweite Haltevorrichtung (143) dazu ausgelegt sind, entlang der Einsteckrichtung (I) zwischen dem Deckelelement (14) und dem ersten Verbinder (1) einen positiven Verriegelungseingriff zu bilden,
**dadurch gekennzeichnet**
**dass** die erste Haltevorrichtung (123) als eine Ausnehmung in einer Wand (125) gebildet ist, die den Fluidkanal (120) umgibt.

2. Verbinderanordnung nach Anspruch 1, **dadurch gekennzeichnet, dass** die erste Haltevorrichtung (123) im Fluidkanal (120) der Einsteckwelle (12) gebildet ist.

3. Verbinderanordnung nach einem der Ansprüche 1 bis 2, **dadurch gekennzeichnet, dass** das Deckelelement (14) einen Deckelkopf (140) umfasst, wobei die zweite Haltevorrichtung (143) entlang der Einsteckrichtung (I) vom Deckelkopf (140) vorsteht.

4. Verbinderanordnung nach einem der vorhergehenden Ansprüche, **dadurch gekennzeichnet, dass** die zweite Haltevorrichtung (143) an einem Ende gegenüber dem Deckelkopf (140) mindestens einen Haltekopf (144) umfasst, und wobei mindestens ein Haltekopf (144) gebildet ist, um den positiven Verriegelungseingriff mit der ersten Haltevorrichtung (123) zu bilden.

5. Verbinderanordnung nach einem der vorhergehenden Ansprüche, **dadurch gekennzeichnet, dass** die Einsteckwelle (12) eine zylindrische oder eine konische Form aufweist, wobei sich der Fluidkanal (120) entlang der Einsteckrichtung (I) in die Einsteckwelle (12) erstreckt.

6. Verbinderanordnung nach einem der vorhergehenden Ansprüche, **dadurch gekennzeichnet, dass** der erste Verbinder (1) einen ersten Körper (10) umfasst, der sich umfänglich um die Einsteckwelle (12) erstreckt, derart, dass radial zwischen dem ersten Körper (10) und der Einsteckwelle (12) ein Innenraum (13) gebildet ist, wobei das Deckelelement (14) einen Einsteckabschnitt (141) umfasst, der in den Innenraum (13) einsteckbar ist.

7. Verbinderanordnung nach Anspruch 6, **dadurch gekennzeichnet, dass** der Einsteckabschnitt (141) konzentrisch zur zweiten Haltevorrichtung (143) angeordnet ist.

8. Verbinderanordnung nach Anspruch 6 oder 7, **dadurch gekennzeichnet, dass** der Einsteckabschnitt (141) radial außerhalb der zweiten Haltevorrichtung (143) angeordnet ist.

9. Verbinderanordnung nach einem der Ansprüche 6 bis 8, **dadurch gekennzeichnet, dass** das Deckelelement (14) dazu ausgelegt ist, den Innenraum (13) zur Außenseite zu schließen, wenn das Deckelelement (14) am ersten Verbinder (1) befestigt ist.

10. Verbinderanordnung nach einem der Ansprüche 6 bis 9, **dadurch gekennzeichnet, dass** der erste Körper (10) zum Miteinanderverbinden des ersten Verbinders (1) und des zweiten Verbinders (2) ein Schraubgewinde (100) umfasst.

11. Verbinderanordnung nach einem der vorhergehenden Ansprüche, **dadurch gekennzeichnet, dass** der erste Verbinder (1) eine erste Verbindungswelle (11) umfasst, die zum Aufnehmen der ersten Verbindungsleitung (3) axial auf die Einsteckwelle (12) ausgerichtet ist.

## Revendications

1. Ensemble raccord pour raccorder des conduites médicales (3, 4) entre elles, comprenant : un premier raccord (1) ayant une tige d'insertion (12) à insérer, selon une direction d'insertion (I), dans une ouverture d'insertion (22) d'un second raccord (2) pour former un raccordement fluidique entre les conduites médicales (3, 4), la tige d'insertion (12) comprenant un canal de fluide (120) permettant au fluide de s'écouler entre le premier raccord (1) et le second raccord (2),
un élément de capuchon (14) pouvant être fixé au premier raccord (1) pour fermer le canal de fluide (120) vers l'extérieur dans un état dans lequel le premier raccord (1) n'est pas raccordé au second raccord (2), le premier raccord (1) ayant un premier dispositif de retenue (123) et l'élément de capuchon (14) ayant un second dispositif de retenue (143), le premier dispositif de retenue (123) et le second dispositif de retenue (143) étant conçus pour former une mise en prise de verrouillage positif entre l'élément de capuchon (14) et le premier raccord (1) le long de la direction d'insertion (I),
**caractérisé en ce**
**que** le premier dispositif de retenue (123) est formé comme un évidement dans une paroi (125) entourant le canal de fluide (120).

2. Ensemble raccord selon la revendication 1, **caractérisé en ce que** le premier dispositif de retenue (123) est formé au sein du canal de fluide (120) de la tige d'insertion (12).

3. Ensemble raccord selon l'une des revendications 1 à 2, **caractérisé en ce que** l'élément de capuchon (14) comprend une tête de capuchon (140), le second dispositif de retenue (143) faisant saillie de la tête de capuchon (140) le long de la direction d'insertion (I).

4. Ensemble raccord selon l'une des revendications précédentes, **caractérisé en ce que** le second dispositif de retenue (143) comprend au moins une tête de retenue (144) au niveau d'une extrémité opposée à la tête de capuchon (140), l'au moins une tête de retenue (144) étant formée pour former ladite mise en prise de verrouillage positif avec le premier dispositif de retenue (123).

5. Ensemble raccord selon l'une des revendications précédentes, **caractérisé en ce que** la tige d'insertion (12) a une forme cylindrique ou conique, le canal de fluide (120) s'étendant le long de la direction d'insertion (I) dans la tige d'insertion (12).

6. Ensemble raccord selon l'une des revendications précédentes, **caractérisé en ce que** le premier raccord (1) comprend un premier corps (10) s'étendant circonférentiellement autour de la tige d'insertion (12) de telle sorte qu'un espace interne (13) est formé radialement entre le premier corps (10) et la tige d'insertion (12), l'élément de capuchon (14) comprenant une partie d'insertion (141) pouvant être insérée dans l'espace interne (13).

7. Ensemble raccord selon la revendication 6, **caractérisé en ce que** la partie d'insertion (141) est agencée de manière concentrique au second dispositif de retenue (143).

8. Ensemble raccord selon la revendication 6 ou 7, **caractérisé en ce que** la partie d'insertion (141) est agencée radialement à l'extérieur du second dispositif de retenue (143).

9. Ensemble raccord selon l'une des revendications 6 à 8, **caractérisé en ce que** l'élément de capuchon (14) est conçu pour fermer l'espace interne (13) vers l'extérieur lorsque l'élément de capuchon (14) est fixé au premier raccord (1).

10. Ensemble raccord selon l'une des revendications 6 à 9, **caractérisé en ce que** le premier corps (10) comprend un filetage (100) pour raccorder le premier raccord (1) et le second raccord (2) l'un à l'autre.

11. Ensemble raccord selon l'une des revendications précédentes, **caractérisé en ce que** le premier raccord (1) comprend une première tige de raccordement (11) alignée axialement avec la tige d'insertion (12) pour recevoir une première conduite de raccordement (3).
